(19) Europäisches Patentamt
European Patent Office
Office européen des brevets

(11) Numéro de publication: **0 188 150**
**A1**

# (12) DEMANDE DE BREVET EUROPEEN

(21) Numéro de dépôt: 85402448.6

(22) Date de dépôt: 10.12.85

(51) Int. Cl.[4]: C 07 D 513/04
A 61 K 31/505, C 07 D 277/18
C 07 B 57/00
//(C07D513/04, 277:00, 239:00)

(30) Priorité: 12.12.84 FR 8418990

(43) Date de publication de la demande: 23.07.86 Bulletin 86/30

(84) Etats contractants désignés: AT BE CH DE FR GB IT LI LU NL SE

(71) Demandeur: RHONE-POULENC SANTE
Les Miroirs 18 Avenue d'Alsace
F-92400 Courbevoie Cedex(FR)

(72) Inventeur: Fabre, Jean-Louis
9, rue Fagon
F-75013 Paris(FR)

(72) Inventeur: Farge, Daniel
30, rue des Pins Sylvestres
F-94320 Thiais(FR)

(72) Inventeur: James, Claude
31 bis, avenue Gambetta
F-75020 Paris(FR)

(74) Mandataire: Le Goff, Yves et al,
RHONE-POULENC RECHERCHES Brevets Pharma 25, Quai Paul Doumer
F-92408 Courbevoie(FR)

**(54) Procédé de préparation d'isomères optiques de thiazolo ]3,2-a[ pyrimidines, les nouveaux isomères ainsi obtenus, les compositions pharmaceutiques qui les contiennent et les intermédiaires pour mettre en oeuvre ce procédé.**

(57) Procédé de préparation d'isomères optiques de produits de formule (I) dans laquelle R = $C_6H_5$, $C_6H_5CH_2$ éventuellement substitué par 1 ou plusieurs halogènes, alcoyle, alcoyloxy, alcoylthio (1 à 4 C) ou bien R = alcoyle (1 à 4 C) non substitué ou substitué par 1 à 3 halogènes, alcényle (2 à 4 C) ou cycloalcoyle (3 à 6 C) et $R_1$ = H ou bien R et $R_1$, différents, représentent $C_6H_5$, $C_6H_5CH_2$ éventuellement substitué par 1 ou plusieurs halogènes, alcoyle, alcoyloxy ou alcoylthio (1 à 4 C) ou bien représentent alcoyle (1 à 4 C) consistant à :

a) ouvrir le produit de formule (I) racémique en produit (II) racémique,

b) faire réagir le produit (II) racémique avec une amine optiquement active pour obtenir un produit (III) sous forme d'un mélange de deux formes diastéréo-isomères,

c) séparer le mélange des deux formes diastéréo-isomères du produit de formule (III),

d) hydrolyser et cycliser séparément chacune de ces formes pour obtenir le produit (I) optiquement actif.

L'invention concerne également les isomères (I) optiquement actifs obtenus et les intermédiaires (II) nécessaires à la mise en oeuvre du procédé.

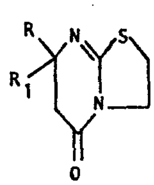

(I)

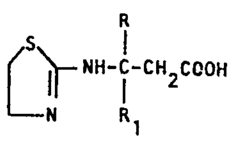

(II)

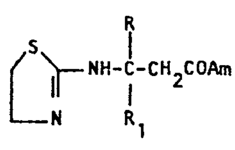

(III)

EP 0 188 150 A1

La présente invention concerne un procédé de préparation d'isomères optiques de thiazolo [3,2-a] pyrimidines, les nouveaux isomères ainsi obtenus, les compositions pharmaceutiques qui les contiennent et les intermédiaires pour mettre en oeuvre le procédé.

Plus précisément, le procédé selon l'invention permet de préparer les isomères optiques de dérivés de la tétrahydro-2,3,6,7 thiazolo [3,2-a] pyrimidinone-5 de formule générale :

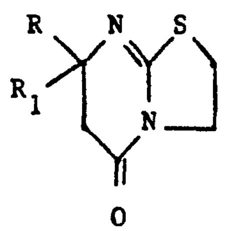

(I)

dans laquelle R représente un radical phényle ou benzyle éventuellement substitué par un ou plusieurs atomes d'halogène ou radicaux alcoyle, alcoyloxy ou alcoylthio ou bien R représente un radical alcoyle contenant 1 à 4 atomes de carbone (non substitué ou substitué par 1 à 3 atomes d'halogène), alcényle contenant 2 à 4 atomes de carbone ou cycloalcoyle contenant 3 à 6 atomes de carbone et $R_1$ représente un atome d'hydrogène, ou bien R et $R_1$, différents, représentent un radical phényle ou benzyle éventuellement substitué par un ou plusieurs atomes d'halogène ou radicaux alcoyle, alcoyloxy ou alcoylthio ou bien représentent un radical alcoyle, étant entendu que les radicaux alcoyle cités ci-dessus ou qui seront cités dans la suite sont en chaîne droite ou ramifiée, ainsi que leurs sels avec les acides pharmaceutiquement acceptables.

La synthèse des produits de formule générale (I) sous forme racémique, à l'exception de ceux pour lesquels R représente un radical benzyle éventuellement substitué comme il est dit précédemment, est décrite dans le brevet européen n° 0045251 mais leur résolution en produits optiquement actifs n'est pas envisagée. Les essais effectués par la demanderesse pour réaliser le dédoublement au moyen des méthodes traditionnelles évidentes pour l'homme du métier (par exemple par formation de sels d'acides optiquement

actifs tels que l'acide camphosulfonique ou dibenzoyltartrique) se sont soldés par des échecs. Ce n'est qu'à la suite de recherches approfondies qu'il a pu être mis au point un procédé indirect d'obtention des formes optiquement actives des produits de formule générale (I) en employant un schéma réactionnel à plusieurs étapes.

Ainsi, selon l'invention, le procédé d'obtention des isomères optiques des produits de formule générale (I) consiste :

a) à ouvrir les produits de formule générale (I) sous forme racémique pour obtenir un acide intermédiaire racémique (en équilibre avec sa forme imine) de formule générale :

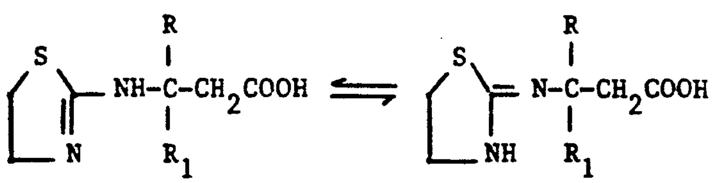

(II)

dans laquelle R et $R_1$ sont définis comme précédemment.

L'ouverture se fait par hydrolyse des produits de formule générale (I) sous forme racémique, de préférence en milieu alcalin en employant par exemple la soude ou la potasse aqueuse à une température comprise entre 20°C et la température de reflux du mélange réactionnel,

b) à faire réagir les produits de formule générale (II) sous forme racémique avec une base organique optiquement active afin d'obtenir un produit (en équilibre avec sa forme imine) de formule générale :

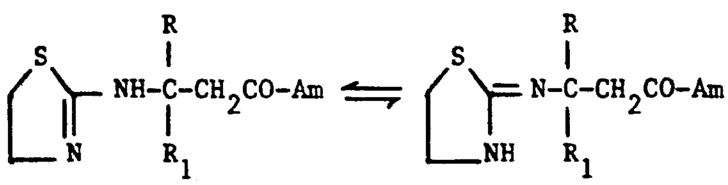

(III)

dans laquelle Am représente un radical amino optiquement actif et R et $R_1$ sont définis comme précédemment, et qui se présente sous forme d'un mélange de deux formes diastéréo-isomères.

La réaction s'effectue généralement dans un solvant organique inerte tel qu'un carbure aromatique (benzène ou toluène) en présence d'hydroxy-2 pyridine, à une température comprise entre

70°C et la température de reflux du mélange réactionnel, en éliminant l'eau formée au cours de la réaction par azéotropie, par exemple au moyen d'un séparateur de Dean et Stark.

Comme base optiquement active correspondant au radical Am défini dans la formule générale (III), on peut citer par exemple l'amino-2 butane, l'amphétamine, l'α-méthylbenzylamine, l'α-(naphthyl-2) éthylamine, la (nitro-4 phényl)-1 éthylamine, la diphényl-1,2 éthylamine, la phényl-2 p-tolyl-1 éthylamine, la phényl-1 p-tolyl-2 éthylamine, l'α-fenchylamine et l'endo-bornylamine. On utilise de préférence une amine primaire, mais il est bien entendu que toute amine optiquement active, primaire ou secondaire, pouvant donner lieu à la formation d'un produit de formule générale (III) convient et entre dans le cadre de la réalisation de la présente invention,

c) à séparer le mélange des deux formes diastéréo-isomères du produit de formule générale (III).

On opère généralement par chromatographie ou par cristallisation des produits à l'état d'acétate en opérant dans un solvant organique tel que l'acétate d'éthyle,

d) à hydrolyser et à cycliser séparément chacune des formes diastéréo-isomères correspondant aux produits de formule générale (III) pour obtenir le produit de formule générale (I) optiquement actif.

On effectue généralement la réaction par chauffage en milieu acide aqueux, par exemple par chauffage en milieu acide chlorhydrique concentré à la température de reflux du mélange réactionnel. Dans ce dernier cas, on isole le produit de formule générale (I) sous forme de chlorhydrate.

Comme on le voit d'après la description des étapes successives de la synthèse, les produits de formule générale (II) sont indispensables à la mise en oeuvre du procédé et forment avec le procédé lui-même un seul concept inventif. De ce fait, ils font partie intégrante de l'invention.

Les nouveaux isomères de formule générale (I) ainsi que les nouveaux intermédiaires de formule générale (II) peuvent être purifiés par les méthodes habituelles connues de l'homme du métier,

en particulier par cristallisation, chromatographie ou passage sur résines échangeuses d'ions suivi éventuellement d'une lyophilisation.

Les nouveaux produits de formule générale (I) et (II) peuvent être transformés éventuellement en sels d'addition avec les acides et les nouveaux produits de formule générale (II) peuvent être transformés en sels métalliques ou en sels d'addition avec les bases azotées par toute méthode connue de l'homme du métier pour effectuer cette transformation sans toucher au reste de la molécule.

Les isomères optiques des produits de formule générale (I) ainsi que leurs sels présentent, comme les racémiques divulgués dans le brevet européen n° 0045251, des propriétés pharmacologiques intéressantes les rendant utiles dans le traitement de fond de la maladie rhumatismale.

Ils sont en particulier actifs à des doses comprises entre 10 et 100 mg/kg par voie orale chez le rat dans l'oedème de la patte provoqué par une réaction d'ARTHUS inversée [oedème provoqué par injection sous-plantaire de 50µg de sérum de lapin antiovalbumine suivie immédiatement par l'injection intraveineuse d'ovalbumine selon la technique de D.K. GEMMELL et Coll., Agents et Actions, 9, 107 (1979)].

Ils sont également actifs chez le rat à des doses généralement comprises entre 5 et 100 mg/kg par jour pendant 7 jours par voie orale vis-à-vis de l'oedème de la patte provoqué par injection sous-plantaire de Bordetella pertussis (Hemophilus pertussis) chez le rat sensibilisé à cet antigène selon la technique de P.A. DIEPPE et Coll., Agents et Actions, 6, 618 (1976).

De plus, les produits de formule générale (I) présentent une faible toxicité. Chez la souris, la toxicité aiguë (exprimée par sa $DL_{50}$) est généralement comprise entre 300 et 900 mg/kg ou supérieure à 900 mg/kg par voie orale.

Les produits de formule générale (II) sont des intermédiaires nouveaux indispensables pour la mise en oeuvre du procédé selon l'invention. En outre, ils présentent en eux-mêmes ainsi que

leurs sels, des propriétés pharmacologiques analogues à celles des produits de formule générale (I). En particulier, ils se sont montrés actifs à des doses voisines de 100 mg/kg par voie orale chez le rat dans l'oedème de la patte provoqué par une réaction d'ARTHUS inversée [oedème provoqué par injection sous-plantaire de 50µg de sérum de lapin antiovalbumine suivie immédiatement par l'injection intraveineuse d'ovalbumine selon la technique de D.K. GEMMELL et Coll., Agents et Actions, 9,107 (1979)].

De plus, les produits de formule générale (II) présentent une faible toxicité. Chez la souris, la toxicité aiguë (exprimée par sa $DL_{50}$) est généralement comprise entre 300 et 900 mg/kg ou supérieure à 900 mg/kg par voie orale.

Pour l'emploi médicinal, il peut être fait usage des nouveaux produits de formule générale (I) tels quels ou à l'état de sels pharmaceutiquement acceptables, c'est-à-dire non toxiques aux doses d'utilisation.

Comme exemples de sels pharmaceutiquement acceptables, on peut citer les sels d'addition avec les acides minéraux tels que chlorhydrates, sulfates, nitrates, phosphates ou organiques tels que acétates, propionates, succinates, benzoates, fumarates, maléates, méthanesulfonates, iséthionates, théophylline-acétates, salicylates, phénolphtalinates, méthylène-bis-β-oxynaphtoates ou des dérivés de substitution de ces composés.

Sont particulièrement intéressants le procédé de préparation des isomères optiques exposé précédemment, les isomères optiques ainsi obtenus et les intermédiaires se rapportant aux produits de formules générales (I) et (II) dans lesquelles R représente un radical méthyle ou un radical benzyle substitué par un atome d'halogène et $R_1$ représente un atome d'hydrogène, plus précisément le procédé et les isomères se rapportant aux produits suivants :

- Méthyl-7 tétrahydro-2,3,6,7 thiazolo [3,2-a] pyrimidinone-5
- p-chlorobenzyl-7 tétrahydro-2,3,6,7 thiazolo [3,2-a] pyrimidinone-5

et les intermédiaires correspondants, c'est-à-dire :

- Acide (Δ2-thiazolinyl-2 amino)-3 butyrique
- Acide (Δ2-thiazolinyl-2 amino)-3 (chloro-4 phényl)-4 butyrique

Les exemples suivants, donnés à titre non limitatif, montrent comment l'invention peut être mise en pratique. Les exemples de référence 1 à 9 montrent comment peuvent être préparés les intermédiaires de formule générale (II) indispensables à la mise en oeuvre du procédé selon l'invention.

EXEMPLE 1

94 g d'acétate de N-(phényl-1' éthyl) [(Δ2-thiazolinyl-2) amino]-3 butyramide-(3R ou S , 1'S) de pouvoir rotatoire $[\alpha]_D^{20}$ = -150,5° (c = 0,5 ; chloroforme) sont dissous dans 190 cm3 d'une solution aqueuse d'acide chlorhydrique 10N et la solution est chauffée au reflux pendant 2 heures et 25 minutes. Après refroidissement, le mélange réactionnel est concentré à sec sous pression réduite (20 mm de mercure ; 2,7 kPa) à une température voisine de 55°C. Le résidu obtenu (111,4 g) est concrété après addition de 150 cm3 d'éthanol. Les cristaux apparus sont séparés par filtration, lavés 2 fois par 40 cm3 au total d'éthanol puis 2 fois par 50 cm3 au total d'éther diéthylique, et séchés sous pression réduite (20 mm de mercure ; 2,7 kPa) en présence de potasse en pastilles. On obtient 17,75 g de produit que l'on dissout dans 860 cm3 d'éthanol bouillant. Après refroidissement à une température voisine de 4°C pendant 48 heures, les cristaux apparus sont séparés par filtration, lavés 1 fois par 50 cm3 d'éthanol puis 1 fois par 50 cm3 d'éther diéthylique et séchés sous pression réduite (20 mm de mercure ; 2,7 kPa) à une température voisine de 20°C. On obtient ainsi 11 g de chlorhydrate de méthyl-7 tétrahydro-2,3,6,7 thiazolo [3,2-a] pyrimidinone-5 lévogyre se sublimant à partir de 220°C et dont le pouvoir rotatoire est $[\alpha]_D^{20}$ = -142° ± 2,6° (c = 0,51 ; eau).

L'acétate de N-(phényl-1' éthyl) [( Δ2-thiazolinyl-2) amino]-3 butyramide-(3 R ou S , 1'S) peut être préparé de la manière suivante :

Un mélange de 71,2 cm3 d'α-méthylbenzylamine-(S) et de 53,3 g d'hydroxy-2 pyridine en solution dans 850 cm3 de toluène est chauffé à 70°C puis additionné de 105,6 g d'acide (Δ2-thiazolinyl-2 amino)-3 butyrique. Le mélange réactionnel est alors chauffé au reflux pendant 5 heures en éliminant l'eau formée au cours de la réaction par entraînement azéotropique au moyen d'un appareil de Dean-Stark. Après refroidissement, l'huile épaisse qui s'est formée est séparée par décantation ; la phase toluénique est concentrée à sec sous pression réduite (20 mm de mercure ; 2,7 kPa) à 60°C. Le résidu obtenu (131,8 g) est chromatographié sur une colonne de 7,4 cm de diamètre contenant 1400 g de silice (0,06 - 0,2 mm). On élue par des mélanges d'acétate d'éthyle et d'éthanol, en recueillant des fractions de 1000 cm3, dans les conditions suivantes :

- Fractions 1 à 4 : (acétate d'éthyle pur)
- Fractions 5 et 6 : (acétate d'éthyle/éthanol : 99/1 en volumes)
- Fractions 7 et 8 : (acétate d'éthyle/éthanol : 98/2 en volumes)
- Fractions 9 et 10 : (acétate d'éthyle/éthanol : 96/4 en volumes)
- Fractions 11 à 37 : (acétate d'éthyle/éthanol : 95/5 en volumes).

Les fractions 1 à 13 sont éliminées ; les fractions 14 à 37 sont réunies et concentrées à sec sous pression réduite (20 mm de mercure ; 2,7 kPa). On obtient ainsi 100,8 g de produit brut que l'on dissout dans 200 cm3 d'acétonitrile bouillant additionné de 1 g de noir décolorant. Après filtration à chaud, le filtrat est refroidi à 4°C pendant 3 heures. Les cristaux apparus sont séparés par filtration, lavés 2 fois par 200 cm3 au total d'éther diéthylique et séchés sous pression réduite (20 mm de mercure ; 2,7 kPa) à une température voisine de 20°C en présence de potasse en pastilles. On obtient ainsi 51,2 g de produit fondant à 116°C. 32 g de produit ainsi préparé sont dissous dans 160 cm3 d'acétate d'éthyle ; la solution est additionnée de 6,3 cm3 d'acide acétique. On amorce la cristallisation puis, après maintien à une température voisine de 20°C pendant 48 heures, les cristaux apparus sont séparés par filtration et séchés sous pression réduite (20 mm de mercure ; 2,7 kPa) à une température voisine de 20°C. On obtient ainsi 11,9 g

d'acétate de N-(phényl-1' éthyl) [( Δ 2-thiazolinyl-2 amino]-3 butyramide-(3 R ou S, 1'S) fondant à 96°C et dont le pouvoir rotatoire est : $[\alpha]_D^{20}$ = -150,5°±2,7° (c = 0,5 ; chloroforme).

L'acide ( Δ 2-thiazolinyl-2 amino)-3 butyrique peut être préparé comme décrit à l'exemple de référence 1.

EXEMPLE 2

22 g d'acétate de N-(phényl-1' éthyl) [(Δ2-thiazolinyl-2) amino]-3 butyramide-(3R ou S , 1'R) de pouvoir rotatoire $[\alpha]_D^{20}$ = + 154° (c = 0,5 ; chloroforme) sont dissous dans 45 cm3 d'une solution aqueuse d'acide chlorhydrique 10N et la solution est chauffée au reflux pendant 105 minutes. Après refroidissement, le mélange réactionnel est concentré à sec sous pression réduite (20 mm de mercure ; 2,7 kPa) à une température voisine de 55°C. Le résidu obtenu est concrété après adddition de 60 cm3 d'éthanol. Les cristaux apparus sont séparés par filtration, lavés par 20 cm3 d'éthanol puis par 40 cm3 d'éther diéthylique et séchés sous pression réduite (20 mm de mercure ; 2,7 kPa) en présence de potasse en pastilles. On obtient ainsi 2 g de produit qui, après addition de 3,2 g de produit préparé de la même manière, sont dissous dans 250 cm3 d'éthanol bouillant. Après refroidissement de la solution à une température voisine de 4°C pendant 24 heures, les cristaux apparus sont séparés par filtration, lavés par 10 cm3 d'éthanol refroidi à une température voisine de 4°C puis par 20 cm3 d'éther diéthylique, et séchés sous pression réduite (20 mm de mercure ; 2,7 kPa) à une température voisine de 20°C en présence de potasse en pastilles. On obtient ainsi 3,5 g de chlorhydrate de méthyl-7 tétrahydro-2,3,6,7 thiazolo [3,2-a] pyrimidinone-5 dextrogyre, se sublimant à partir de 220°C et dont le pouvoir rotatoire est : $[\alpha]_D^{20}$ = + 139,7° ± 2,7° (c = 0,5 ; eau).

L'acétate de N-(phényl-1' éthyl) [ Δ 2-thiazolinyl-2) amino]-3 butyramide-(3R ou S , 1'R) peut être préparé de la manière suivante :

Un mélange de 54,5 cm3 d'α-méthylbenzylamine-(R) et de 40,8 g d'hydroxy-2 pyridine en solution dans 550 cm3 de toluène est chauffé à 70°C puis additionné de 80,7 g d'acide (Δ2-thiazolinyl-2 amino)-3 butyrique. Le mélange réactionnel est alors chauffé au reflux pendant 3 heures en éliminant l'eau formée au cours de la réaction par entraînement azéotropique au moyen d'un appareil de Dean-Stark. Après refroidissement, un insoluble est séparé par filtration, lavé 2 fois par 80 cm3 au total de toluène et éliminé. Les filtrats sont réunis et concentrés à sec sous pression réduite (20 mm de mercure ; 2,7 kPa) à 60°C. Le résidu obtenu (168,5 g) est chromatographié sur une colonne de 8,4 cm de diamètre contenant 1700 g de silice (0,06-0,2 mm). On élue par des mélanges d'acétate d'éthyle et d'éthanol, en recueillant des fractions de 1000 cm3 dans les conditions suivantes :

- Fractions 1 à 6 : (acétate d'éthyle pur)
- Fractions 7 à 10 : (acétate d'éthyle/éthanol : 98/2 en volumes)
- Fractions 11 à 14 : (acétate d'éthyle/éthanol : 95/5 en volumes)
- Fractions 15 à 18 : (acétate d'éthyle/éthanol : 90/10 en volumes)
- Fractions 18 à 21 : (acétate d'éthyle/éthanol : 95/15 en volumes)

Les fractions 1 à 13 sont éliminées ; les fractions 14 à 21 sont réunies et concentrées à sec sous pression réduite (20 mm de mercure ; 2,7 kPa). On obtient ainsi 116,2 g de produit brut que l'on dissout dans 150 cm3 d'acétate d'éthyle bouillant. Après refroidissement à 4°C pendant 16 heures, les cristaux apparus sont séparés par filtration, lavés 1 fois avec 80 cm3 d'acétate d'éthyle, 1 fois par 60 cm3 d'oxyde d'isopropyle et séchés sous pression réduite (20 mm de mercure ; 2,7 kPa) à une température voisine de 20°C en présence de potasse en pastilles. On obtient ainsi 35 g de produit fondant à 119°C. Ce produit est dissous dans 175 cm3 d'acétate d'éthyle. Après addition de 6,85 cm3 d'acide acétique, on chauffe jusqu'à une température voisine de 70°C, puis refroidit à une température voisine de 20°C, filtre un léger insoluble résiduel et lave avec 20 cm3 d'acétate d'éthyle. Les filtrats sont réunis ; on amorce la cristallisation puis, après maintien à une température

voisine de 20°C pendant 48 heures, les cristaux apparus sont séparés par filtration, lavés 1 fois par 15 cm3 d'acétate d'éthyle, et séchés sous pression réduite (20 mm de mercure ; 2,7 kPa) à une température voisine de 20°C. On obtient ainsi 8 g de produit fondant à 93°C. Ce produit est dissous dans 25 cm3 d'acétate d'éthyle bouillant. Après refroidissement à une température voisine de 4°C pendant 48 heures, les cristaux apparus sont séparés par filtration, lavés successivement par 10 cm3 d'acétate d'éthyle puis par 15 cm3 d'oxyde d'isopropyle, et séchés sous pression réduite (20 mm de mercure ; 2,7 kPa) à une température voisine de 20°C. On obtient ainsi 7,4 g d'acétate de N-(phényl-1' éthyl) [(Δ 2-thiazolinyl-2) amino]-3 butyramide-(3 R ou S, 1'R) fondant à 95°C et dont le pouvoir rotatoire est : $[\alpha]_D^{20}$ = +154° ± 2,7° (c = 0,5 ; chloroforme).

L'acide (Δ 2-thiazolinyl-2 amino)-3 butyrique peut être préparé comme décrit à l'exemple de référence 1.

EXEMPLE 3

60 mg de N-(phényl-1' éthyl) (chloro-4 phényl)-4 [(Δ2-thiazolinyl-2) amino]-3 butyramide-(3R ou S, 1'R) de pouvoir rotatoire $[\alpha]_D^{20}$= + 37°C (c = 0,5; chloroforme) sont dissous dans 5 cm3 d'une solution aqueuse d'acide chlorhydrique 10N et la solution est chauffée au reflux pendant 90 minutes. Après refroidissement, le mélange réactionnel est concentré à sec sous pression réduite (20 mm de mercure; 2,7 kPa) à une température voisine de 55°C. Le résidu obtenu est concrété après addition de 5 cm3 d'éther diéthylique. Les cristaux apparus sont séparés par filtration,

lavés par 2 cm3 d'éther diéthylique et séchés sous pression réduite (20 mm de mercure; 2,7 kPa) en présence de potasse en pastilles. On obtient ainsi 10 mg de produit qui sont dissous dans 0,1 cm3 d'éthanol bouillant. Après refroidissement de la solution à une température voisine de 4°C pendant 24 heures, les cristaux apparus sont séparés par filtration, lavés par 0,1 cm3 d'éthanol refroidi à une température voisine de 4°C puis par 0,5 cm3 d'éther diéthylique, et séchés sous pression réduite (20 mm de mercure; 2,7 kPa) à une température voisine de 20°C en présence de potasse en pastilles. On obtient ainsi 1,6 mg de chlorhydrate de (chloro-4 benzyl)-7 tétrahydro-2,3,6,7 thiazolo [3,2-a] pyrimidinone-5 dextrogyre, se sublimant à partir de 166°C et dont le pouvoir rotatoire est :
$[\alpha]_D^{20} = +77,4° \pm 10,0°$(c = 0,062; eau).

Le N-(phényl-1' éthyl) (chloro-4 phényl)-4 [Δ 2-thiazolinyl-2) amino]-3 butyramide-(3R ou S, 1'R) peut être préparé de la manière suivante :

Un mélange de 1,03 cm3 d'α-méthylbenzylamine-(R) et de 0,78 g d'hydroxy-2 pyridine en solution dans 10 cm3 de toluène est chauffé à 70°C puis additionné de 2,4 g d'acide (chloro-4 phényl)-4 (Δ2-thiazolinyl-2 amino)-3 butyrique. Le mélange réactionnel est alors chauffé au reflux pendant 3 heures en éliminant l'eau formée au cours de la réaction par entraînement azéotropique au moyen d'un appareil de Dean-Stark. Après refroidissement, un insoluble est séparé par filtration, lavé 2 fois par 5 cm3 au total de toluène et éliminé. Les filtrats sont réunis et concentrés à sec sous pression réduite (20 mm de mercure; 2,7 kPa) à 60°C. Le résidu obtenu (4 g) est chromatographié sur une colonne de 4 cm de diamètre contenant 150 g de silice (0,040-0,063 mm). On élue par un mélange d'acétate d'éthyle et de méthanol (90/10 en volumes) sous une pression de 0,5 bar (51 kPa) et en recueillant des fractions de 50 cm3.

Les fractions 1 à 5 sont éliminées; les fractions 6 et 7 sont réunies et concentrées à sec sous pression réduite (20 mm de mercure; 2,7 kPa). On obtient ainsi 0,3 g de produit brut que l'on dissout dans 1,1 cm3 d'acétate d'éthyle bouillant. Après refroidissement à 4°C pendant 30 minutes, les cristaux apparus sont séparés par filtration, lavés 1 fois avec 0,1 cm3 d'acétate d'éthyle, 1 fois par 2 cm3 d'oxyde d'isopropyle et séchés sous pression réduite (20 mm de mercure; 2,7 kPa) à une température voisine de 20°C en présence de potasse en pastilles. On obtient ainsi 0,08 g de N-(phényl-1' éthyl) (chloro-4 phényl)-4 [(Δ2-thiazolinyl-2) amino]-3 butyramide-(3 R ou S, 1'R) fondant à 148°C et dont le pouvoir rotatoire est : $[\alpha]_D^{20}$ = +37,0°± 2,4° (c = 0,5; chloroforme).

L'acide (chloro-4 phényl)-4 (Δ 2-thiazolinyl-2 amino)-3 butyrique peut être préparé comme décrit à l'exemple de référence 9.

EXEMPLE DE REFERENCE 1

450 g de méthyl-7 tétrahydro-2,3,6,7 thiazolo [3,2-a] pyrimidinone-5 sont dissous dans 1600 cm3 de propanol-2 par chauffage à 50°C. Après dissolution complète, on ajoute 265 cm3 d'une solution aqueuse à 30% de soude et on chauffe à la température de reflux du mélange réactionnel pendant 10 minutes. Le mélange réactionnel est alors concentré à sec sous pression réduite (20 mm de mercure ; 2,7 kPa) à 40°C. Le résidu obtenu est repris avec 500 cm3 de propanol-2 et concentré à nouveau. Cette opération est répétée 2 fois puis le résidu obtenu est dissous dans 900 cm3 de propanol-2 bouillant. Après refroidissement à une température voisine de 20°C pendant 18 heures, les cristaux apparus sont séparés par filtration, lavés 3 fois par 900 cm3 au total de propanol-2 et séchés sous pression réduite (1 mm de mercure ; 1,35 kPa) à une température voisine de 20°C en présence de potasse en pastilles. On obtient ainsi 467 g de sel de sodium de l'acide (Δ2-thiazolinyl-2 amino)-3 butyrique sous forme de cristaux blancs fondant à 204°C. 172 g du sel de sodium ainsi préparé sont dissous dans 820 cm3 d'eau distillée et la solution ainsi obtenue est chargée sur une colonne contenant 1700 cm3 de résine échangeuse d'ions DOWEX 50X-2 (50-100 mesh ; U.S. Standard ASTM E 11-61) sous forme acide. Les produits neutres sont élués successivement avec 1500 cm3 d'eau distillée, 500 cm3 de méthanol, et 1500 cm3 d'eau distillée. Le produit attendu est alors élué avec 5500 cm3 d'une solution aqueuse à 2,5% de pyridine. Après lyophilisation de la solution ainsi obtenue, on obtient 149,3 g d'un solide blanc que l'on dissout dans 250 cm3 d'éthanol à 95% bouillant. Après refroidissement à une température voisine de 4°C pendant 18 heures, les cristaux apparus sont séparés par filtration, lavés une fois avec 20 cm3 d'éthanol glacé puis 3 fois par 75 cm3 au total d'oxyde d'isopropyle et séchés sous pression réduite (20 mm de mercure ; 2,7 kPa) à une température voisine de 20°C en présence de potasse en pastilles. On obtient ainsi 120,5 g d'acide (Δ2-thiazolinyl-2 amino)-3 butyrique sous forme de cristaux blancs fondant à 125°C.

La méthyl-7 tétrahydro-2,3,6,7 thiazolo [3,2-a] pyrimidinone-5 peut être préparée comme décrit dans le brevet européen n° 0045251.

EXEMPLE DE REFERENCE 2

En opérant de manière analogue à celle décrite à l'exemple de référence 1 mais à partir de 66 g d'éthyl-7 tétrahydro-2,3,6,7 thiazolo [3,2-a] pyrimidinone-2, on obtient 37 g d'acide (Δ2-thiazolinyl-2 amino)-3 valérique, sous forme de cristaux blancs fondant à 120°C.

L'éthyl-7 tétrahydro-2,3,6,7 thiazolo [3,2-a] pyrimidinone-5 peut être préparée comme décrit dans le brevet européen n° 0045251.

EXEMPLE DE REFERENCE 3

En opérant de manière analogue à celle décrite à l'exemple de référence 1 mais à partir de 7,9 g d'isopropyl-7 tétrahydro-2,3,6,7 thiazolo [3,2-a] pyrimidinone-5, on obtient, après lyophylisation, 7,6 g d'un solide blanc que l'on réunit avec 2,1 g de produit préparé de la même manière dans une opération antérieure. La purification de ces 9,7 g est effectuée par chromatographie sur colonne sèche suivant la technique décrite apr B. LOEV et M.M. GOODMAN, Progr. Separ. Purif., 1970, 3, 73-95, à l'aide de 100 g de silice (0,063-0,2 mm) en utilisant l'acétate d'éthyle comme solvant. Le produit attendu est récupéré par extraction à l'aide de 100 cm3 d'eau suivie d'une lyophilisation. On obtient ainsi 7,8 g d'un solide blanc qui, après dissolution complète dans 50 cm3 de méthanol à 20°C, reprécipite. Les cristaux apparus sont séparés par filtration, lavés 3 fois par 50 cm3 au total d'éther diéthylique et séchés sous pression réduite (20 mm de mercure ; 2,7 kPa) à une température voisine de 20°C en présence de potasse en pastilles. On obtient ainsi 7,05 g d'acide (Δ 2-thiazolinyl-2 amino)-3 méthyl-4 pentanoïque, sous forme de cristaux blancs fondant à 228°C.

L'isopropyl-7 tétrahydro-2,3,6,7 thiazolo [3,2-a] pyrimidinone-5 peut être préparée comme décrit dans le brevet européen n° 0045251.

EXEMPLE DE REFERENCE 4

8,3 g de n-propyl-7 tétrahydro-2,3,6,7 thiazolo [3,2-a] pyrimidinone-5 sont dissous dans 42 cm3 d'une solution aqueuse de soude 1N. La solution obtenue est maintenue pendant 1 heure à une température voisine de 20°C puis est lyophilisée. La laque jaune ainsi obtenue est alors concrétée avec 40 cm3 d'acétone. Les cristaux apparus sont séparés par filtration, lavés 1 fois par 10 cm3 d'acétone et 1 fois par 40 cm3 d'éther diéthylique et séchés sous pression réduite (20 mm de mercure ; 2,7 kPa) à une température voisine de 20°C en présence de potasse en pastilles. On obtient ainsi 9,3 g de sel de sodium de l'acide (Δ2-thiazolinyl-2 amino)-3 hexanoïque, sous forme de cristaux blancs fondant à 218°C.

La n-propyl-7 tétrahydro-2,3,6,7 thiazolo [3,2-a] pyrimidinone-5 peut être préparée comme décrit dans le brevet européen n° 0045251.

EXEMPLE DE REFERENCE 5

6,4 g d'isobutyl-7 tétrahydro-2,3,6,7 thiazolo [3,2-a] pyrimidinone-5 sont mis en suspension dans 30 cm3 d'eau distillée. On ajoute alors 30 cm3 d'une solution aqueuse de soude 1N. Après dissolution, le louche résiduel est éliminé par filtration. Le filtrat est alors lyophilisé. On obtient ainsi 7,9 g de produit brut, sous forme de meringue blanche que l'on cristallise par reprise à l'aide de 30 cm3 d'acétone. Après refroidissement à une température voisine de 5°C, les cristaux apparus sont séparés par filtration, lavés 3 fois par 60 cm3 au total d'éther diéthylique, et séchés sous pression réduite (20 mm de mercure ; 2,7 kPa) à une température voisine de 20°C en présence de potasse en pastilles. On

obtient ainsi 7,3 g de sel de sodium de l'acide (Δ2- thiazolinyl-2 amino)-3 méthyl-5 hexanoïque, sous forme de cristaux blancs fondant à 228°C.

L'isobutyl-7 tétrahydro-2,3,6,7 thiazolo [3,2-a] pyrimidinone-5 peut être préparée comme décrit dans le brevet européen n° 0045 251.

EXEMPLE DE REFERENCE 6

En opérant de manière analogue à celle décrite à l'exemple de référence 5, mais à partir de 7,15 g de cyclohexyl-7 tétrahydro-2,3,6,7 thiazolo [3,2-a] pyrimidinone-5, de 30 cm3 de solution aqueuse de soude 1N et de 25 cm3 d'eau distillée, on obtient 8,3 g de sel de sodium de l'acide (Δ2-thiazolinyl-2 amino)-3 cyclohexyl-3 propionique, sous forme de cristaux blancs fondant à 272°C.

Le cyclohexyl-7 tétrahydro-2,3,6,7 thiazolo [3,2-a] pyrimidinone-5 peut être préparée comme décrit dans le brevet européen n° 0045251.

EXEMPLE DE REFERENCE 7

6,7 g de (propène-1 yl)-7 tétrahydro-2,3,6,7 thiazolo [3,2-a] pyrimidinone-5 sont mis en suspension dans 34 cm3 d'une solution aqueuse de soude 1N. Après 24 heures d'agitation, le résidu insoluble est éliminé par filtration. Le filtrat est lyophilisé. On obtient ainsi 8,1 g de produit brut que l'on met en suspension pendant 2 heures dans 60 cm3 de chloroforme. Le solide est alors séparé par filtration, lavé 2 fois par 20 cm3 au total de chloroforme puis trituré dans 50 cm3 d'acétone. Les cristaux apparus sont séparés par filtration, lavés avec 25 cm3 d'acétone et séchés sous pression réduite (1 mm de mercure ; 0,135 kPa) à une température voisine de 20°C. On obtient ainsi 3,35 g de sel de sodium de l'acide (Δ 2-thiazolinyl-2 amino)-3 hexène-4 oïque (mélange des formes Z et E), sous forme de cristaux blancs fondant à 180°C.

La (propèn-1 yl)-7 tétrahydro-2,3,6,7 thiazolo [3,2-a] pyrimidinone-5 peut être préparée comme décrit dans le brevet européen n° 0045251.

EXEMPLE DE REFERENCE 8

9,28 g de phényl-7 tétrahydro-2,3,6,7 thiazolo [3,2-a] pyrimidinone-5 sont mis en suspension dans 40 cm3 d'une solution aqueuse de soude 1N. Après dissolution, le louche résiduel est éliminé par filtration. Le filtrat est alors lyophilisé. On obtient ainsi 11,15 g de produit brut qui, après addition de 2,7 g de produit préparé de la même façon, sont triturés pendant 2 heures dans 70 cm3 d'acétone. Les cristaux apparus sont séparés par filtration, lavés 2 fois par 40 cm3 au total d'acétone puis 2 fois par 50 cm3 au total d'éther diéthylique, et séchés sous pression réduite (1 mm de mercure ; 0,135 kPa) à une température voisine de 20°C en présence de potasse en pastilles. Les cristaux ainsi obtenus sont alors maintenus à l'air libre jusqu'à stabilisation du poids. On obtient ainsi 13,15 g de monohydrate du sel de sodium de l'acide (Δ 2-thiazolinyl-2 amino)-3 phényl-3 propionique sous forme de cristaux blancs fondant à 251°C.

La phényl-7 tétrahydro-2,3,6,7 thiazolo [3,2-a] pyrimidinone-5 peut être préparée comme décrit dans le brevet européen n° 0045251.

EXEMPLE DE REFERENCE 9

En opérant de manière analogue à celle décrite à l'exemple de référence 1 mais à partir de 2,35 g de p-chlorobenzyl-7 tétrahydro-2,3,6,7 thiazolo [3,2-a] pyrimidinone-5 et de 0,84 cm3 d'une solution aqueuse à 30% de soude, on obtient 0,22 g d'acide (chloro-4 phényl)-4 (Δ 2-thiazolinyl-2 amino)-3 butyrique, sous forme de cristaux blancs fondant à 102°C.

La p-chlorobenzyl-7 tétrahydro-2,3,6,7 thiazolo [3,2-a] pyrimidinone-5 peut être préparée de la manière suivante :

Une solution de 0,9 g de chlorure de (chloro-4 phényl)-4 chloro-3 butyryle dans 2 cm3 de toluène et une solution de 1,13 g d'amino-2 thiazoline-2 dans 10 cm3 de dioxanne anhydre sont ajoutées goutte à goutte simultanément en 5 minutes à 2 cm3 de toluène préalablement chauffés au reflux, puis le mélange réactionnel est chauffé au reflux pendant 45 minutes. Après refroidissement à 20°C environ, les cristaux apparus sont séparés par filtration et éliminés. Le filtrat est concentré à sec sous pression réduite (20 mm de mercure ; 2,7 kPa) à 60°C et le résidu (1,5 g) est chromatographié sur une colonne de 1,1 cm de diamètre contenant 15 g d'alumine neutre (0,12 - 0,15 mm) en éluant par un mélange de chloroforme et de cyclohexane (80/20 en volumes) et en recueillant des fractions de 5 cm3. Les 4 premières fractions sont réunies et concentrées à sec sous pression réduite (20 mm de mercure ; 2,7 kPa) à 60°C. On obtient ainsi 0,4 g de produit que l'on chromatographie à nouveau sur une colonne de 1,1 cm de diamètre contenant 4 g d'alumine neutre (0,12-0,15 mm) en éluant par un mélange de chloroforme et de cyclohexane (50/50 en volumes) et en recueillant des fractions de 3 cm3. Les 2 premières fractions sont éliminées. Les 7 fractions suivantes sont réunies et concentrées à sec sous pression réduite (20 mm de mercure ; 2,7 kPa) à 60°C. On obtient ainsi 0,17 g de produit que l'on chromatographie une troisième fois sur une colonne de 1,1 cm de diamètre contenant 6 g d'alumine neutre (0,12-0,15 mm) en éluant par un mélange de chloroforme et de cyclohexane (50/50 en volumes) et en recueillant des fractions de 3 cm3. On obtient ainsi 0,1 g de laque jaune que l'on dissout dans 1 cm3 d'éthanol. Après addition de 0,08 cm3 d'une solution 4,66N d'acide chlorhydrique gazeux dans l'éther, refroidissement à une température voisine de 4°C pendant 1 heure, et addition de 1 cm3 d'éther, les cristaux apparus, sont séparés par filtration, lavés 1 fois par 1 cm3 d'un mélange éthanol-éther diéthylique (50/50 en volumes) et 1 fois par 1 cm3 d'un mélange éthanol-éther diéthylique (20/80 en volumes) puis séchés sous pression réduite (20 mm de mercure ; 2,7 kPa) à une température

voisine de 20°C en présence de potasse en pastilles. On obtient finalement 0,072 g de p-chlorobenzyl-7 tétrahydro-2,3,6,7 thiazolo [3,2-a] pyrimidinone-5 à l'état de chlorhydrate, sous forme de cristaux blancs fondant à 197°C.

Le chlorure de (chloro-4 phényl)-4 chloro-3 butyryle peut être préparé de la façon suivante :

0,8 g d'acide (chloro-4 phényl)-4 hydroxy-3 butyrique en solution dans 8 cm3 de chloroforme et 0,25 cm3 de diméthylformamide sont additionnés goutte à goutte de 0,54 cm3 de chlorure de thionyle en 2 minutes, puis chauffés progressivement au reflux jusqu'à la fin du dégagement gazeux. Le mélange réactionnel est alors concentré par distillation du chloroforme et de l'excès de chlorure de thionyle sous pression réduite (20 mm de mercure ; 2,7 kPa). Après reprise à l'aide de 10 cm3 de toluène et nouvelle concentration sous pression réduite (20 mm de mercure ; 2,7 kPa), on obtient 0,9 g de chlorure de (chloro-4 phényl)-4 chloro-3 butyryle sous forme d'une huile marron.

L'acide (chloro-4 phényl)-4 hydroxy-3 butyrique peut être préparé selon la méthode décrite par D.I. BARRON et coll., J. Med. Chem., 11, 1139-44 (1968).

La présente invention concerne également les médicaments constitués par au moins un produit de formule générale (I), à l'état d'isomère optique pur, tel quel, ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale ou rectale.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

En thérapeutique humaine, les produits de l'invention sont particulièrement utiles dans le traitement de fond de la maladie rhumatismale et des états allergiques. Les doses dépendent de l'effet recherché et de la durée du traitement ; elles sont généralement comprises entre 50 et 1000 mg par jour par voie orale pour un adulte en une ou plusieurs prises.

D'une façon générale, le médecin déterminera la posologie qu'il estime la plus appropriée en fonction de l'âge, du poids et de tous les autres facteurs propres au sujet à traiter.

L'exemple suivant, donné à titre non limitatif, illustre une composition selon l'invention.

EXEMPLE A

On prépare selon la technique habituelle des comprimés dosés à 50 mg de produit actif ayant la composition suivante :

- Méthyl-7 tétrahydro-2,3,6,7 5H-thiazolo [3,2-a] pyrimidinone-5, lévogyre .................. 50 mg
- amidon .............................................. 15 mg
- silice colloïdale ................................ 9,5 mg
- stéarate de magnésium ............................ 0,5 mg

EXEMPLE B

On prépare selon la technique habituelle des comprimés dosés à 50 mg de produit actif ayant la composition suivante :

- (p-chlorobenzyl)-7 tétrahydro-2,3,6,7 5H-thiazolo [3,2-a] pyrimidinone-5, dextrogyre .................. 50 mg
- amidon .............................................. 15 mg
- silice colloïdale ................................ 9,5 mg
- stéarate de magnésium ............................ 0,5 mg

# REVENDICATIONS

1 - Procédé de préparation d'isomères optiques de thiazolo [3,2-a] pyrimidines de formule générale :

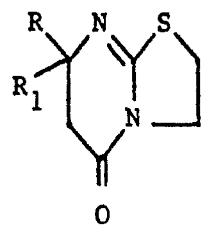

(I)

dans laquelle R représente un radical phényle ou benzyle éventuellement substitué par un ou plusieurs atomes d'halogène ou radicaux alcoyle, alcoyloxy ou alcoylthio ou bien R représente un radical alcoyle contenant 1 à 4 atomes de carbone (non substitué ou substitué par 1 à 3 atomes d'halogène), alcényle contenant 2 à 4 atomes de carbone ou cycloalcoyle contenant 3 à 6 atomes de carbone et $R_1$ représente un atome d'hydrogène, ou bien R et $R_1$, différents, représentent un radical phényle ou benzyle éventuellement substitué par un ou plusieurs atomes d'halogène ou radicaux alcoyle, alcoyloxy ou alcoylthio ou bien représentent un radical alcoyle, étant entendu que les radicaux alcoyle sont en chaîne droite ou ramifiée, caractérisé en ce qu'il consiste :

a) à ouvrir un produit de formule générale (I) sous forme racémique pour obtenir un acide intermédiaire racémique (en équilibre avec sa forme imine) de formule générale :

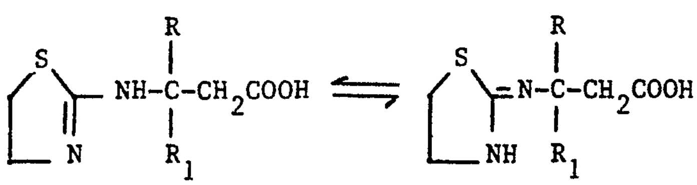

(II)

dans laquelle R et $R_1$ sont définis comme précédemment,

b) à faire réagir le produit de formule générale (II) sous forme racémique avec une base organique optiquement active afin d'obtenir un produit de formule générale :

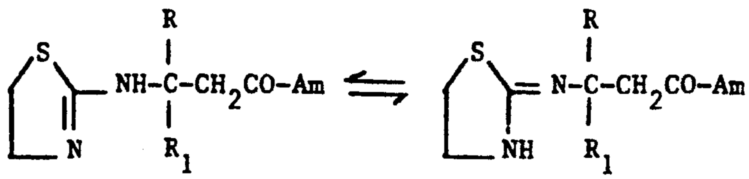

(III)

dans laquelle Am représente un radical amino optiquement actif et R et $R_1$ sont définis comme précédemment, et qui se présente sous forme d'un mélange de deux formes diastéréo-isomères,

c) à séparer le mélange des deux formes diastéréo-isomères du produit de formule générale (III),

d) à hydrolyser et à cycliser séparément chacune des formes diastéréoisomères correspondant au produit de formule générale (III) pour obtenir le produit de formule générale (I) optiquement actif puis à isoler le produit obtenu et à le transformer éventuellement en un sel avec un acide pharmaceutiquement acceptable.

2 - Procédé selon la revendication 1, caractérisé en ce que l'ouverture du produit de formule générale (I) racémique en produit de formule générale (II) racémique s'effectue par hydrolyse alcaline.

3 - Procédé selon la revendication 1, caractérisé en ce que l'amine optiquement active correspondant au radical Am dans la formule générale (III) est une amine primaire.

4 - Procédé selon les revendications 1 et 3, caractérisé en ce que l'amine optiquement active correspondant au radical Am dans la formule générale (III) est choisie parmi l'amino-2 butane, l'amphétamine, l'α-méthylbenzylamine, l'α-(naphtyl-2) éthylamine, la (nitro-4 phényl)-1 éthylamine, la diphényl-1,2 éthylamine, la phényl-2 p-tolyl-1 éthylamine, la phényl-1 p-tolyl-2 éthylamine, l'α-fenchylamine et l'endo-bornylamine.

5 - Procédé selon l'une des revendications 1, 3 ou 4, caractérisé en ce que l'amine optiquement active correspondant au radical Am dans la formule générale (III) est l'α-méthyl benzylamine.

6 - Procédé selon la revendication 1, caractérisé en ce que l'on sépare les deux formes diastéréo-isomères des produits de formule générale (III) sous forme d'acétate.

7 - Isomères optiques de tétrahydro-2,3,6,7 thiazolo [3,2-a] pyrimidinone-5 de formule générale :

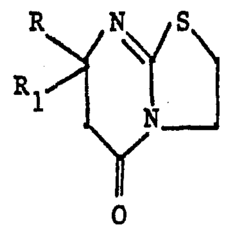

(I)

dans laquelle R représente un radical phényle ou benzyle éventuellement substitué par un ou plusieurs atomes d'halogène ou radicaux alcoyle, alcoyloxy ou alcoylthio ou bien R représente un radical alcoyle contenant 1 à 4 atomes de carbone (non substitué ou substitué par 1 à 3 atomes d'halogène), alcényle contenant 2 à 4 atomes de carbone ou cycloalcoyle contenant 3 à 6 atomes de carbone et $R_1$ représente un atome d'hydrogène, ou bien R et $R_1$, différents, représentent un radical phényle ou benzyle éventuellement substitué par un ou plusieurs atomes d'halogène ou radicaux alcoyle, alcoyloxy ou alcoylthio ou bien représentent un radical alcoyle contenant 1 à 4 atomes de carbone, étant entendu que les radicaux alcoyle cités ci-dessus sont en chaîne droite ou ramifiée, ainsi que leurs sels avec les acides pharmaceutiquement acceptables.

8 - Composition pharmaceutique, caractérisée en ce qu'elle contient au moins un produit selon la revendication 7 en association avec un ou plusieurs diluants ou adjuvants compatibles et pharmaceutiquement acceptables.

9 - Produit intermédiaire nécessaire à la mise en oeuvre du procédé selon la revendication 1, caractérisé en ce qu'il répond à la formule générale suivante se trouvant en équilibre avec sa forme imine :

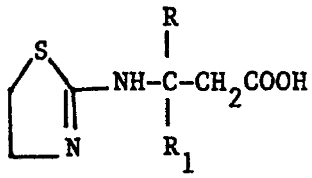

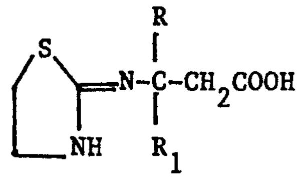

(II)

dans laquelle R représente un radical phényle ou benzyle éventuellement substitué par un ou plusieurs atomes d'halogène ou radicaux alcoyle, alcoyloxy ou alcoylthio ou bien R représente alcoyle contenant 1 à 4 atomes de carbone, (non subtitué ou substitué par 1 à 3 atomes d'halogène), alcényle contenant 2 à 4 atomes de carbone ou cycloalcoyle contenant 3 à 6 atomes de carbone et $R_1$ représente un atome d'hydrogène, ou bien R et $R_1$, différents, représentent un radical phényle ou benzyle éventuellement substitué par un ou plusieurs atomes d'halogène ou radicaux alcoyle, alcoyloxy ou alcoylthio ou bien représentent un radical alcoyle contenant 1 à 4 atomes de carbone, étant entendu que les radicaux alcoyle cités ci-dessus sont en chaîne droite ou ramifiée.

R E V E N D I C A T I O N S

1 - Procédé de préparation d'isomères optiques de thiazolo [3,2-a] pyrimidines de formule générale :

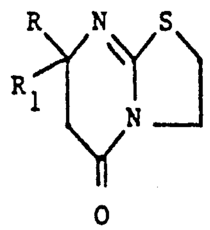

(I)

dans laquelle R représente un radical phényle ou benzyle éventuellement substitué par un ou plusieurs atomes d'halogène ou radicaux alcoyle, alcoyloxy ou alcoylthio ou bien R représente un radical alcoyle contenant 1 à 4 atomes de carbone (non substitué ou substitué par 1 à 3 atomes d'halogène), alcényle contenant 2 à 4 atomes de carbone ou cycloalcoyle contenant 3 à 6 atomes de carbone et $R_1$ représente un atome d'hydrogène, ou bien R et $R_1$, différents, représentent un radical phényle ou benzyle éventuellement substitué par un ou plusieurs atomes d'halogène ou radicaux alcoyle, alcoyloxy ou alcoylthio ou bien représentent un radical alcoyle, étant entendu que les radicaux alcoyle sont en chaîne droite ou ramifiée, caractérisé en ce qu'il consiste :

a) à ouvrir un produit de formule générale (I) sous forme racémique pour obtenir un acide intermédiaire racémique (en équilibre avec sa forme imine) de formule générale :

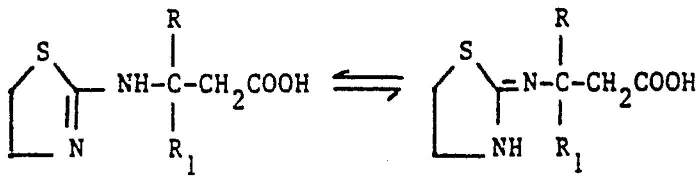

(II)

dans laquelle R et $R_1$ sont définis comme précédemment,

b) à faire réagir le produit de formule générale (II) sous forme racémique avec une base organique optiquement active afin d'obtenir un produit de formule générale :

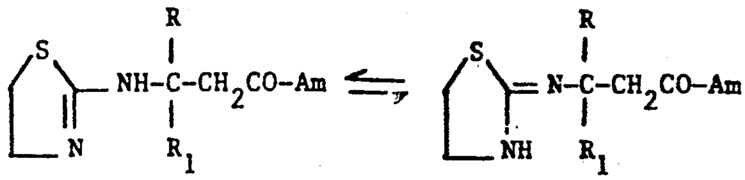

(III)

dans laquelle Am représente un radical amino optiquement actif et R et $R_1$ sont définis comme précédemment, et qui se présente sous forme d'un mélange de deux formes diastéréo-isomères,

c) à séparer le mélange des deux formes diastéréo-isomères du produit de formule générale (III),

d) à hydrolyser et à cycliser séparément chacune des formes diastéréoisomères correspondant au produit de formule générale (III) pour obtenir le produit de formule générale (I) optiquement actif puis à isoler le produit obtenu et à le transformer éventuellement en un sel avec un acide pharmaceutiquement acceptable.

2 - Procédé selon la revendication 1, caractérisé en ce que l'ouverture du produit de formule générale (I) racémique en produit de formule générale (II) racémique s'effectue par hydrolyse alcaline.

3 - Procédé selon la revendication 1, caractérisé en ce que l'amine optiquement active correspondant au radical Am dans la formule générale (III) est une amine primaire.

4 - Procédé selon les revendications 1 et 3, caractérisé en ce que l'amine optiquement active correspondant au radical Am dans la formule générale (III) est choisie parmi l'amino-2 butane, l'amphétamine, l'α-méthylbenzylamine, l'α-(naphtyl-2) éthylamine, la (nitro-4 phényl)-1 éthylamine, la diphényl-1,2 éthylamine, la phényl-2 p-tolyl-1 éthylamine, la phényl-1 p-tolyl-2 éthylamine, l'α-fenchylamine et l'endo-bornylamine.

5 - Procédé selon l'une des revendications 1, 3 ou 4, caractérisé en ce que l'amine optiquement active correspondant au radical Am dans la formule générale (III) est l'α-méthyl benzylamine.

REVENDICATIONS SPECIALES AUTRICHE



6 - Procédé selon la revendication 1, caractérisé en ce que l'on sépare les deux formes diastéréo-isomères des produits de formule générale (III) sous forme d'acétate.

7 - Procédé de préparation d'un produit intermédiaire nécessaire à la mise en oeuvre du procédé selon la revendication 1, de formule générale suivante se trouvant en équilibre avec sa forme imine :

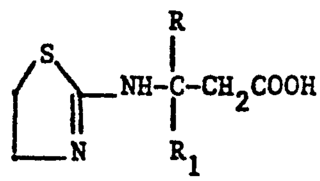

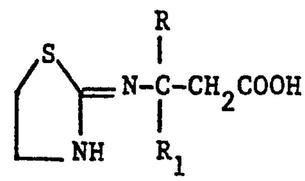

(II)

dans laquelle R représente un radical phényle ou benzyle éventuellement substitué par un ou plusieurs atomes d'halogène ou radicaux alcoyle, alcoyloxy ou alcoylthio ou bien R représente alcoyle contenant 1 à 4 atomes de carbone, (non subtitué ou substitué par 1 à 3 atomes d'halogène), alcényle contenant 2 à 4 atomes de carbone ou cycloalcoyle contenant 3 à 6 atomes de carbone et $R_1$ représente un atome d'hydrogène, ou bien R et $R_1$, différents, représentent un radical phényle ou benzyle éventuellement substitué par un ou plusieurs atomes d'halogène ou radicaux alcoyle, alcoyloxy ou alcoylthio ou bien représentent un radical alcoyle contenant 1 à 4 atomes de carbone, étant entendu que les radicaux alcoyle cités ci-dessus sont en chaîne droite ou ramifiée, caractérisé en ce qu'il consiste à ouvrir un produit de formule générale :

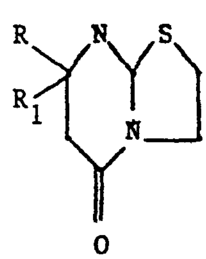

(I)

REVENDICATIONS SPECIALES AUTRICHE



sous forme racémique dans laquelle R représente un radical phényle ou benzyle, éventuellement substitué par un ou plusieurs atomes d'halogène ou radicaux alcoyle, alcoyloxy ou alcoylthio ou bien R représente un radical alcoyle contenant 1 à 4 atomes de carbone (non substitué ou substitué par 1 à 3 atomes d'halogène), alcényle contenant 2 à 4 atomes de carbone ou cycloalcoyle contenant 3 à 6 atomes de carbone et $R_1$ représente un atome d'hydrogène, ou bien R et $R_1$, différents, représentent un radical phényle ou benzyle éventuellement substitué par un ou plusieurs atomes d'halogène ou radicaux alcoyle, alcoyloxy ou alcoylthio ou bien représentent un radical alcoyle, étant entendu que les radicaux alcoyle sont en chaîne droite ou ramifiée, puis isole le produit obtenu.

Office européen des brevets

**0188150**

Numéro de la demande

# RAPPORT DE RECHERCHE EUROPEENNE

EP 85 40 2448

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int. Cl.4) |
| D,A | EP-A-0 045 251 (RHONE-POULENC) * Revendications 1,6; page 4, lignes 7-10 * ----- | 7,8 | C 07 D 513/04<br>A 61 K 31/505<br>C 07 D 277/18<br>C 07 B 57/00 //<br>(C 07 D 513/04<br>C 07 D 277:00<br>C 07 D 239:00) |
| | | | DOMAINES TECHNIQUES RECHERCHES (Int. Cl.4) |
| | | | C 07 D 513/00<br>A 61 K 31/00<br>C 07 D 277/00 |

Le présent rapport de recherche a été établi pour toutes les revendications

| Lieu de la recherche | Date d'achèvement de la recherche | Examinateur |
|---|---|---|
| LA HAYE | 19-03-1986 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant